# EUROPEAN PATENT APPLICATION

(11) **EP 1 146 121 A1**
(43) Date of publication of application: **17.10.2001**
(21) Application number: 00201363.9
(22) Date of filing: 14.04.2000
(51) Int. Cl.: C12N 9/10, C12P 21/02, C12P 13/02, C07K 5/037, C07K 14/00, A61K 38/45

(54) **Method for modification of proteins by transglutaminase**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Boumans, Johannes Wilhelmus Leonardus, 1191 RH Ouderkerk aan de Amstel (NL); Wijbenga, Dirk-Jan, 9301 WZ Roden (NL); Bos, Harmannus Theodorus Petrus, 9781 BG Bedum (NL); Wijngaards, Gerrit, 3972 GS Driebergen-Rijsenburg (NL); Heeres, André, 9715 AE Groningen (NL)
(74) Representative: Wittop Koning, Tom Hugo

(57) **Abstract**

The invention relates to a method for coupling a peptide or protein bound glutamine residue to a substrate by acyl transfer, catalysed by a transglutaminase activity whereby the substrate is chosen from the group, consisting of lysine-free peptides of at least 2 amino acids, lysine-free proteins, aminosaccharides, saccharides, aminopolysaccharides and alcohols. In particular, the method can be used to confer to a peptide comprising a glutamine residue the capacity to keep its activity upon passing the stomach. Further the use of transglutaminase for epitope masking is described.

## Description

The present invention relates to a novel method for enzymatic modification of proteins, especially to a method for coupling a peptide or protein bound glutamine residue by acyl transfer, catalysed by a transglutaminase activity. The term "peptide or protein bound glutamine" refers to an amino acid sequence (i.e. a peptide or a protein), wherein the glutamine residue is at least two amino acids spaced away from the N-terminus of the said amino acid sequence.

In the art, transglutaminase is known to catalyse an acyl transfer reaction, wherein the γ-carboxamine group of glutaminyl residues is the acyl donor. Substrates that can act as the acyl acceptor are less defined and can roughly be divided into three groups, wich will be discussed below.

First, the ε-aminogroup of protein bound lysine residues can serve as substrate, leading to the generation of intra- and intermolecular β-(γ-glutamyl)-lysine isopeptide bonds. In the art, transglutaminase is used to couple a glutamine comprising peptide or protein to lysine residues of a protein, in order to change certain properties of the said protein, such as improvement of the texture of a protein food product, for improvement of the solubility of a protein etc. (for a review, see Nielsen, Food Biotechnology, 9(3), 119-156 (1995)). However, the said proteins should have sufficient suitable lysine residues as acyl acceptors. Whereas the peptide or protein must comprise at least a suitable glutamine residue as acyl donor.

Second, specific primary amines, lysine or ethyl esters of particular amino acids are described as acceptors of the acyl group in the transglutaminase catalysed reaction. In this case, the acyl donors are peptide or protein bound glutaminyl residues. Ikura et al. (Agric. Bio.Chem, 45 (11), 2587 - 2592, 1981) describes the use of transglutaminase for the incorporation of methionine ethyl ester into food proteins, wherein the amine group of the amino acid ethyl ester is coupled to the δ carbon of protein bound glutamine residues. By this method, the food proteins could be enriched with limiting essential amino acids by covalent attachment thereof to the said protein. In this way, food proteins of altered amino acid composition could be produced.

Third, water is described to act as the acyl acceptor, a reaction which is pH dependent (see Nielsen, supra). Water as acceptor in the transglutaminase catalysed acyl transfer reaction results in the conversion of glutamine into glutamic acid, therewith altering the amino acid composition of the protein as well as its functional properties.

The present inventors have now discovered novel properties and uses of the transglutaminase enzyme. It was surprisingly found that transglutaminase can catalyse the acyl transfer reaction between peptide or protein bound glutamine and a number of novel substrates. Therefore, the invention provides a method for coupling a peptide or protein bound glutamine residue to a substrate by acyl transfer, catalysed by a transglutaminase activity, charactarized in that the substrate is chosen from the group, consisting of lysine-free peptides of at least 2 amino acids, lysine-free proteins, aminosaccharides, aminopolysaccharides, saccharides and alcohols.

"Transglutaminase activity" is defined as the activity of a compound to catalyse an acyl transfer reaction, wherein the γ-carboxamide group of glutamine residues is the acyl donor. Said glutaminase activity therefore comprises the transglutaminases that are presently known in the art, which are widely distributed in nature and have been isolated from e.g. mammals, plants, fish and micro-organisms.

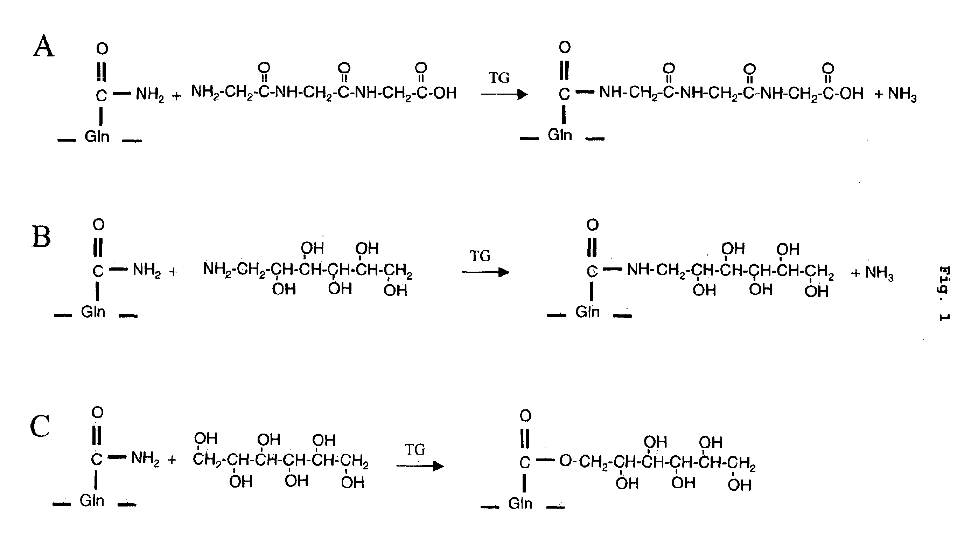

It was found that the amine group of the amino terminal of peptides of at least 2 amino acids could be used as acyl acceptor in the above-mentioned transglutaminase catalysed reaction, leading to a protein, one or more of the glutamine residues thereof being bound to the peptides. This reaction is illustrated in fig. 1.

In an attractive embodiment of the invention, the substrate is chosen from peptides and proteins, the N-terminal thereof being preferably glycine, alanine, serine or valine, more preferably glycine. As will be explained in the following examples, such peptides could successfully be used by several transglutaminases and could be coupled to the glutamyl residues of a protein. When the N-terminal amino acid of the peptide is glycine, a very versatile peptide is obtained that can be coupled to a peptide or protein bound glutamine by several tested transglutaminases. The said coupling reaction has been found to be improved when the second amino acid from the N-terminus of the substrate is chosen from glycine, alanine and valine, but the best coupling was obtained when the second amino acid from the N-terminal of the peptide was glycine. More preferably, the substrate comprises at least 3 amino acids.

In a preferred embodiment of the invention, the substrate is a peptide of 3-20 amino acids, although longer amino acid chains can be used as well. The said substrates can be coupled to one or more glutamines of the protein or peptide, therewith altering the properties of the peptide/protein in a desired manner. As outlined above, the person, skilled in the art, will be aware of the possibilities for improving certain qualities or activities of proteins and peptides by coupling to one or more of the glutamine residues thereof a substrate according to the present invention.

In another embodiment of the invention, the substrate is an aminosaccharide. This reaction is illustrated in fig. 1. "Aminosaccharides" are defined as any monosaccharide, oligosaccharide or polysaccharide containing a primary amino group and any monosaccharide, oligosaccharide or polysaccharide prepared by means of reductive amination of a monosaccharide, oligosaccharide or polysaccharide. An example of an aminosaccharide is aminosorbitol. "Saccharides" are defined as any reducing monosaccharide, oligosaccharide or polysaccharide, such as starch hydrolysates, enzymatic prepared oligosaccharide syrops, cellulose and b-glucans.

In a preferred embodiment, the peptide or protein bound glutamyl residue is part of a biologically functional peptide or protein or functional fragment thereof. As is outlined above, the coupling of one or more of the substrates according to the invention will lead to a peptide or protein or fragment thereof having improved function, quality or activity. The biologically functional peptide or protein or functional fragment thereof may be a natural peptide, protein or fragment, but may also be a manipulated peptide, protein or fragment, e.g. by genetic manipulation techniques.

In an attractive embodiment, the peptide or protein bound glutamine residue is part of an epitope. "Epitope" is defined as an amino acid sequence that forms a specific binding site for an immunoglobuline molecule. By coupling this glutamine residue to a substrate according to the present invention, the said epitope may be masked, by which the protein can loose its antigenic activity. It is also possible that the glutamyl residue is in close vicinity of an epitope, e.g. 1 to 10 amino acids spaced therefrom; when a sufficient long or bulky substrate is coupled to the said glutamine residue, the said epitope can be successfully masked. The epitope, or the surrounding amino acid sequence, may also be engineered in such a way, that it comprises one or more glutamines on a position where it is not present in the natural counterpart of the said epitope, in order to couple, at the said glutamines, the substrates according to the invention.

In another embodiment, the transglutaminase activity comprises a transglutaminase of microbial origin or a functional derivative thereof. As will be shown below, transglutaminase of microbial origin has a versatile utility with respect of the method according to the present invention. Microbial transglutaminase is e.g. capable of successfully couple peptides of two or three amino acids to a peptide or protein bound glutamine residue. Transglutaminase, derived from guinea pig liver is however also suitable, although the versatility thereof appears to be somewhat less than that of microbial origin. Preferably, the calcium independent transglutaminase of *Streptoverticillium sp.* is used, more preferably from *Streptoverticillium mobaraense* (Ando et al. Agric. Biol. Chem., 53(10), 2613-17, 1989). The transglutaminase of the said microorganism has been shown very versatile for the method according to the present invention and has the additional advantage that no calcium is necessary for enzymatic activity thereof. For a review of calcium-independent microbial transglutaminase, see Zhu et al., Appl. Microbiol. Biotechnol. (1995) 44:277-282.

The invention further relates to a method as described above, for conferring to the peptide/protein comprising the glutamine residue, the capacity to substantially keep its activity upon passing the stomach of a mammal, which mammal preferably is human. Many functional peptides and proteins are degraded upon passing the stomach and therewith loosing their activity. In order to maintain the activity, one or more of the glutamine residues of the said peptide or protein is coupled to a substrate according to the present invention.

On the other hand, the N-terminus of peptides or proteins is often susceptible to degradation in the stomach. In order to avoid degradation of the said peptides/proteins, the N-terminus thereof can be coupled to a glutamine comprising carrier peptide/protein, using transglutaminase according to the invention. Thus, the N-terminus of the peptide/protein to be stabilised is coupled to a glutamine residue of the carrier, resulting in protection of the N-terminus of the stabilised peptide/protein. The person, skilled in the art will know which substrates or carriers are to be coupled to the protein in order to obtain the desired resistance against degradation. Examples of proteins that can serve as carrier are gluten proteins, soy proteins and milk proteins. Examples of peptides to be stabilised are bio-active peptides, in particular anti-microbial peptides which are effective in the gastrointestinal tract.

The invention further relates to a method as described above for masking an epitope of a peptide/protein. Transglutaminase is used to covalently link one or more of the abovementioned substrates to a glutamine that is part of the epitope or located in close vicinity thereof, but according to the invention it may also be used to covalently link a lysine within the epitope or in close vicinity thereof to a glutamine of a masking peptide or protein. By this method, the said peptide/protein is rendered less antigenic. Preferably, the epitope comprises a glutamine, or a glutamine is in close vicinity of the said epitope.

The invention also relates to a peptide or protein obtainable by the method according to the invention, i.e. to proteins and peptides, one or more glutamine residues of which are coupled to one or more of the substrates that are discussed above. To this end, the invention relates to a peptide or protein comprising at least a glutamine residue, the δ-carbon thereof being covalently linked to a substrate, the substrate being chosen from:
- a second lysine-free peptide or lysine-free protein, at least comprising 2 amino acids, the free α-aminogroup thereof being covalently linked to the δ-carbon of the glutamine residue.
- an aminosaccharide or aminopolysaccharide, the aminogroup thereof being covalently linked to the δ-carbon of the glutamine residue.
- a saccharide, polysaccharide or alcohol, a hydroxygroup thereof being covalently linked to the δ-carbon of the glutamine residue.
Preferably the substrate is chosen from peptides and proteins, the N terminal thereof preferably being as described above.

### Figures

Figure 1 is a schematic diagram of the acyl transfer reaction from a γ-carboxamide group of a peptide or protein bond glutaminyl residue, catalysed by transglutaminase, with different substrates as acyl acceptor. A: the tripeptide gly-gly-gly as substrate, B: the aminosaccharide aminosorbitol as substrate, C: sorbitol as substrate.
Fig. 2. MALDI-TOF analysis of dB1 before (A) and after modification with TG and gly-gly-gly (B).
Fig. 3. SDS-PAGE analysis of dB1 incubated with TG. Samples were taken after different time points as indicated at the top of each lane. Lane 2 shows unmodified dB1.
Fig. 4. (A) SDS-PAGE analysis of α-lactalbumin incubated with TG and with or without gly-gly-gly. Samples were taken at time points indicated at the top of each lane. Due to cross-linking by TG, protein polymers in lanes 5 and 6 at the interface of the stacking and running gel are too large to enter the running gel. (B) NH₃ production of the time samples dipicted as molecules NH₃ per molecules of α-lactalbumin.
Fig. 5. Structure of 1-amino-1-deoxy-D-sorbitol (A), 1-amino Paselli (R₁=(glu)ₙ) (B) and 1-amino-1-deoxy-dextran (R₂=(glu)ₙ) (C) .

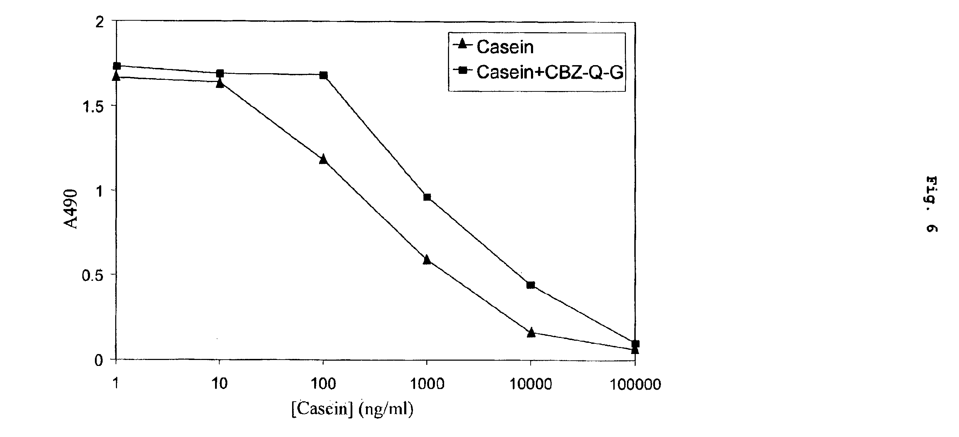

Fig. 6. Inhibition ELISA of casein and casein modified with CBZ-gln-gly (CBZ-Q-G) peptides using microbial TG.
The invention will be further illustrated by the following examples which are not intended to limit the scope thereof.

### Example 1

### Incorporation of peptides into dB1

The dB1 protein is a model protein that corresponds with a part of the central domain of high molecular weight (HMW) wheat gluten and contains a high number of glutamines but no lysines. This makes the protein a suitable substrate to study incorporation of primary amines and deamidation catalysed by transglutaminase, since no glutamine-lysine cross-linking can occur.

A large number of peptides were incubated with methylated dB1 (mdB1) and TG (mdB1 1 mg/ml, peptide 1 mg/ml, TG 0,2 U/ml, 37°C, 4 hours) and the products were analysed using MALDI-TOF, a method that determines the mass of molecules with great precision (Hillenkamp and Ehring, in Mass Spectrometry in the Biological Sciences (1992) M.L. Gross (Eds) Kluwer Academic, Dordrecht, The Netherlands). Fig. 1 shows incorporation of glycine-tripeptide into mdB1. Peaks correspond to mdB1 with varying numbers of glycine-tripeptide incorporated, the number being indicated. Thus, incorporation of this peptide via coupling of the α-amino group occurs to a high extent, as roughly half of the 64 glutaminyl residues present in mdB1 can be coupled. (Increased TG concentration and extended incubation time increased the number to a maximum of 31 peptides incorporated.)

To study the effect of adjacent amino acids on the ability of TG to use the α-amino group as a substrate different peptides were analysed in the same experiment as for glycine-tripeptide. First, the third amino acid was changed to study the effect of size and charge of its side chain (see Table 1). In order to quantify the numbers of peptides incorporated we took the highest peak in the MALDI-TOF spectrum as the average value. As shown in Table 1, an increased size of the third amino acid is related to a somewhat slower rate of incorporation. However, a more profound effect can be seen in case of a charged amino acid. The negative charge of glutamic acid results in a significantly lower level of incorporation, whereas that for the peptide with positive charge at this position is almost the same as for the glycine-tripeptide.

**TABLE 1.**

| Numbers of incorporation of peptides into mdB1. | | | |
|---|---|---|---|
| Peptide | # peptides/mdB1 | Peptide | # peptides/mdB1 |
| gly-gly-gly | 19 | ala | 0 |
| gly-gly-ala | 14 | val | 0 |
| gly-gly-val | 10 | ala-gly-gly | 1 |
| gly-gly-ile | 11 | val-gly-gly | >0 |
| gly-gly-glu | 6 | ser-gly-gly | 0,5 |
| gly-gly-arg | 16 | ile-gly-gly | >0 |
| gly-gly | 13 | tyr-gly-gly | >0 |
| gly-ala | 8 | arg-gly-gly | >0 |
| gly-val | 7 | gln-gly | >0 |
| gly-glu | 0.5 | asp-gly | 0 |
| gly-tyr | 1.5 | gly-OEt | 28 |
| gly | >0 | gly-gly-OEt | 22 |

It is postulated that decreased reactivity of the α-amino group is related to the vicinity of the C-terminal negative charge of the peptide. This explains why the glycine-dipeptide is significantly less reactive than the tripeptide and that glycine is almost not reactive at all. It also explains the charge effects observed with the charged third amino acid: glutamic acid at this position results in an increase of the negative charge at the C-terminus, whereas the positive charge of the side chain of arginine compensates for it. In fact, since the gly-gly-arg peptide is incorporated at almost the same rate as the glycine-tripeptide, despite the bulkiness of the arginine, it can be concluded that the effect of charge is more relevant to reactivity than that of size. It can therefore be hypothesized that in case of large peptides increased size of side chains is less relevant than suggested by the numbers in Table 1. Further evidence for the importance of the C-terminal charge is found when glycine-ethylester (gly-Oet) and glycine-glycine-ethylester (gly-gly-Oet) were used as substrates. Changing the second amino acid gives similar results as for the third amino acid. Even when the amino acid at the N-terminus is changed, incorporation is observed, although to a relatively low extent. Thus, the α-amino group of peptides can serve as a substrate for transglutaminase and the presence of a glycine residue at the N-terminus has been shown to be important for a high rate of incorporation into proteins.

### Example 2

### Incorporation of bio-active peptides into dB1

Using similar experiments as described in Example 1, several bio-active peptides were incorporated into dB1 (see Table 2). These results indicate that the length of the peptide is not limited to those used in example 1. Furthermore, the results indicate that gluten proteins can serve as a carrier for bio-active peptides. In this way these peptides may keep their activity upon passing the stomach.

**TABLE 2.**

| Bio-active peptides incorporated into dB1 | | |
|---|---|---|
| Name | Sequence | Function |
| Amyloid peptide | GAIIGLMVGGVV | Induce fusion of liposomes in vitro |
| Dynorphin | GGFLRRI | Potentiates the dorsal immobility response in rats |
| Enkephalinase inhibitor | GGFM | Inhibitor of enkephalinase |
| Neuromedin B | GNLWATGHFM-NH₂ | A potent stimulant effect on the smooth muscle preparation of rat uterus |

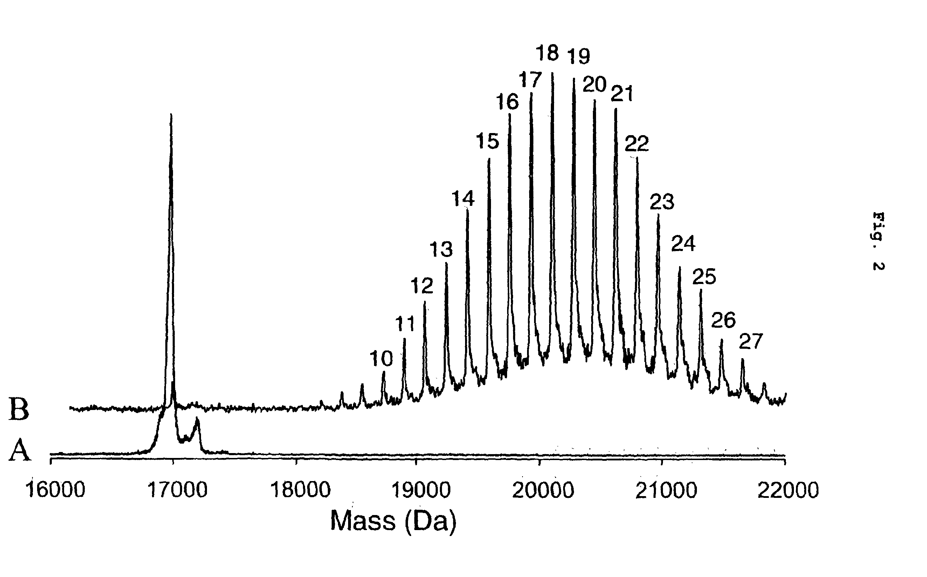

Analysis of dB1 incubated with TG on SDS-PAGE indicates that polymerisation occurs, despite the fact that this protein does not contain any lysine residues (see Fig. 2). Analysis by Edman-degradation indicated that the amount of accessible N-terminus of dB1 modified by TG is reduced by a factor of 10 compared to the same amount of unmodified dB1. This shows that coupling occurs via the amine group at the N-terminus of the protein. The experiment thus shows that coupling of the N-terminal amine group to a glutamine residue is not limited to peptides, but can also link entire proteins.

### Example 3

### Incorporation of glycine-tripeptide into α-lactalbumin

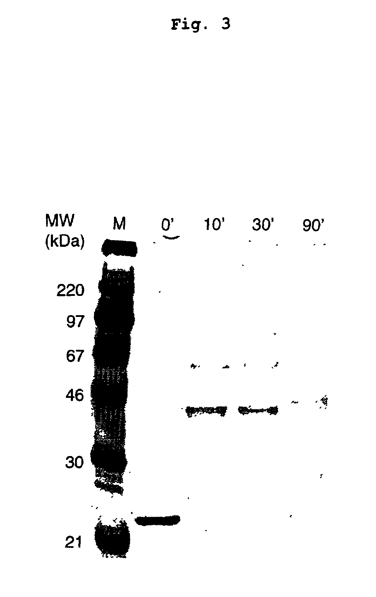

To be able to make a comparison between the reaction rates of crosslinking versus incorporation of peptides α-lactalbumin was incubated with TG and with or without glycine-tripeptide (Fig. 3). As shown in Fig. 3A, protein crosslinking is largely inhibited by the presence of glycine-tripeptide, strongly suggesting that the rate of the incorporation reaction is significantly higher than that of the crosslinking reaction. This is supported by Fig. 3B, which shows the NH₃ production of the two reactions in time. Since NH₃ is a product in all types of reactions catalysed by TG, production of NH₃ represents a summation of protein crosslinking, incorporation and deamidation. Previous experiments with several protein substrates have shown that the reaction rate of deamidation is significantly slower than that of protein crosslinking. Based on SDS-PAGE analysis and measurement of NH₃ release it can be concluded that when glycine-tripeptide is present the incorporation reaction represents to over 90% of the total reaction rate. Mass analyses using MALDI-TOF indicates that a maximum of five incorporations can be achieved, even after prolonged incubation with higher concentrations of TG (data not shown). This suggests that one of the 6 glutaminyl residues in α-lactalbumin is not accessible for the incorporation reaction. It can, however, not be excluded that this remaining residue is accessible for deamidation.

### Example 4

### Incorporation of aminosaccharides into dB1

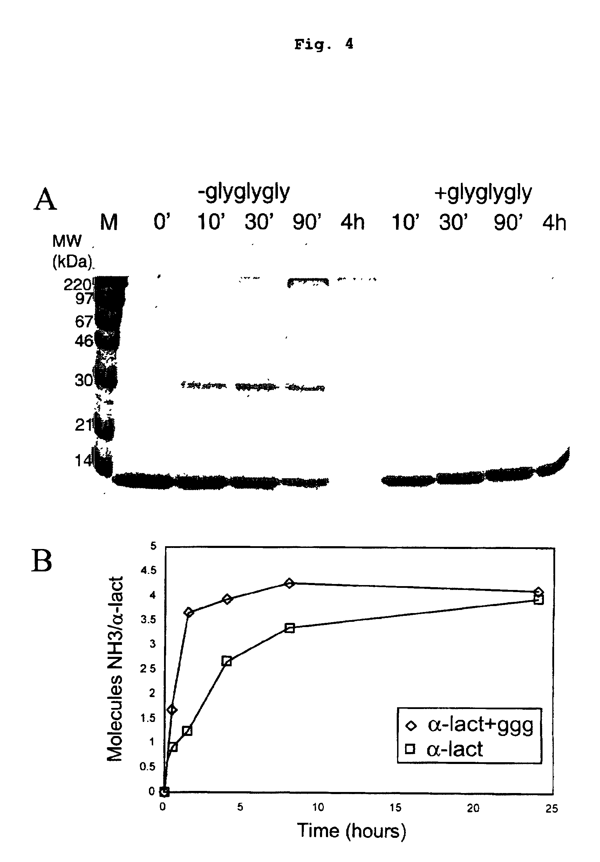

The structure of 1-amino-1-deoxy-D-sorbitol (aminosorbitol) is depicted in Fig. 4. This compound was incubated with dB1 and TG similar to the experiments described in example 1. Surprisingly, aminosorbitol could be incorporated into dB1 as could be shown by MALDI-TOF and NH₃ production (data not shown). This incorporation is relatively efficient, since up to 10 molecules of aminosorbitol per dB1 could be coupled.

The finding that a hydroxylated amino-alkylchain can act as the acyl acceptor prompted us to investigate the possibilities to incorporate amino-oligosaccharides. In order to study this type of reaction, we used the model peptide CBZ-glutamine-glycine instead of dB1. Using MALDI-TOF, coupling of aminodextran MW 1500 and amino MD20 Paselli to CBZ-gln-gly could be demonstrated. Reactivity of the latter indicates that a spacer of 4 (hydroxylated) C atoms between the amino group and the bulk of the oligosaccharide is sufficient for coupling (see Fig. 4).

### Example 5

### Incorporation of sorbitol and alcohols into dB1

Sorbitol, methanol and propanol were incubated with dB1 and microbial TG under conditions described in Example 1 and the results were analysed by MALDI-TOF. It was shown that incorporation occurs with all three substrates, although to a very low extent. Since none of these molecules contains an amine group it implies that the hydroxyl group, although a much weaker nucleophile, is also able to act as the acyl acceptor. This type of reaction thus resembles that of the deamidation reaction, in which water acts as the acyl acceptor.

### Example 6

### Incorporation of peptides using mammalian transglutaminases

Transglutaminases from different sources exhibit different substrate specificity. We therefore examined whether bovine blood coagulation Factor XIII and guinea pig liver transglutaminase are capable of incorporating the peptides mentioned in Table 1 into mdB1. Factor XIII did not show any incorporation of the glycine-tripeptide into mdB1, showing that the α-amino group is not a substrate for this enzyme. When the guinea pig liver enzyme was used, glycine-tripeptide was incorporated to about the same extend as when used microbial TG. However, when peptides were used with amino acids increasing in size, the level of incorporation rapidly decreased (data not shown). This shows that although the α-amino group can serve as a substrate for guinea pig liver TG, the active site of the enzyme draws more limitations to the size of the side chains of the amino acids than the microbial TG does. This is further shown by the fact that tyrosinaminde can be incorporated by the microbial TG, but not using the enzyme from guinea pig liver. Furthermore, the reactions described in Example 5 were also tested using Factor XIII and guinea pig liver transglutaminase, but no incorporation could be detected.

### Example 7

### Reduction of protein allergenity by modification using transglutaminase

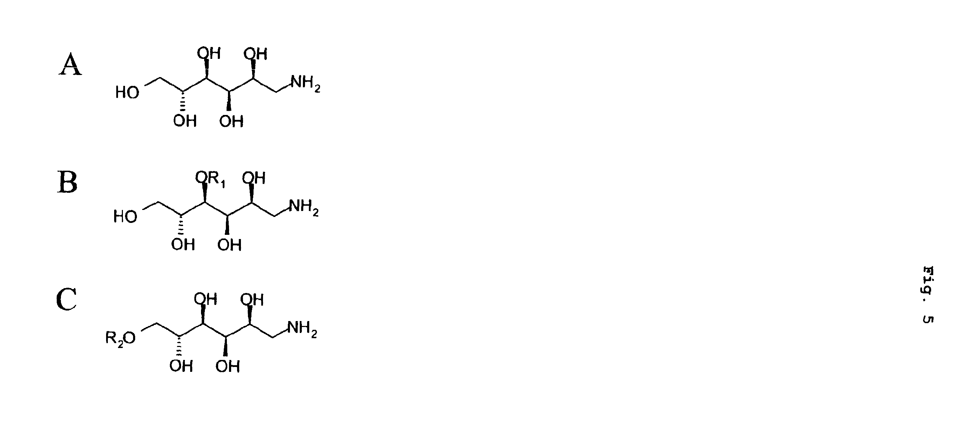

As a source of IgE directed against casein, serum form 6 patients with a casein allergy was used. Affinities of casein for IgE were measured using IgE binding experiments as described earlier for another allergen (Koppelman et al. J. Biol. Chem. 274, 4770-7, 1999). Dilutions of casein (final concentrations 1 to 100000 ng/ml), either or not modified with mTG and CBZ-gln-gly under conditions described in Example 1, were incubated with a 1 to 30 dilution of patient serum pool in phosphate buffered saline (PBS) containing 1% BSA and 0.1% Tween 20. In this fluid phase, casein was allowed to bind to IgE for one hour at room temperature under gently shaking conditions. In order to determine the non-bound IgE fraction, the incubation mixtures were transferred to the 96-well plates pre-treated as follows. 96-well plates were coated with 10 µg/ml native casein in PBS and subsequently blocked with BSA (1% in PBS containing 0.1% Tween 20) to diminish the non-specific binding. IgE bound to the casein coated wells was detected using an anti-human IgE antibody conjugated to horse radish peroxidase. Between each step, plates were washed five times with PBS containing 0.1% Tween 20. The inhibition of IgE binding as a function of the amount of casein present in the pre-incubation sample reflects the affinity of casein for IgE. The concentrations needed for half-maximal inhibition were calculated using a semi-logarithmic equation and were used to compare the affinities of the different forms of casein for IgE. Fig. 5 shows that the affinity of mTG/CBZ-gln-gly treated casein for IgE was decreased 5 times compared to native casein.

## Claims

1. Method for coupling a peptide or protein bound glutamine residue to a substrate by acyl transfer, catalysed by a transglutaminase activity, charactarized in that the substrate is chosen from the group, consisting of lysine-free peptides of at least 2 amino acids, lysine-free proteins, aminosaccharides, saccharidess, aminopolysaccharides and alcohols.

2. Method according to claim 1, wherein the substrate is chosen from peptides and proteins, the N-terminal thereof being glycine, alanine, valine or serine, preferably glycine.

3. Method according to claim 2, wherein the second amino-acid from the N-terminus of the substrate is glycine, alanine, valine, preferably glycine.

4. Method according to any of the preceding claims, **characterized in that** the substrate comprises a peptide of 3-20 amino acids.

5. Method according to claim 1, **characterized in that** the substrate is an aminosaccharide.

6. Method according to any of the preceding claims, wherein the peptide or protein bound glutamine residue is part of a biologically functional peptide or protein or functional fragment thereof.

7. Method according to claim 6, wherein the peptide or protein bound glutamine residue is part of or in close vicinity of an epitope.

8. Method according to any of the preceding claims, wherein the transglutaminase activity comprises a transglutaminase enzyme of microbial origin or a functional derivative thereof, preferably being derived from *Streptoverticillium mobaraense*.

9. Method according to any of the previous claims for conferring to the peptide/protein comprising the glutamine residue the capacity to substantially keep its activity upon passing the stomach of a mammal, preferably human.

10. Method according to any of the claims 1-8 for masking an epitope of the peptide/protein comprising the glutamine residue.

11. Peptide or protein, comprising at least a glutamine residue, the δ-carbon thereof being covalently linked to a substrate, the substrate being chosen from:
- a second lysine-free peptide or lysine-free protein, at least comprising 2 amino acids, the free α-aminogroup thereof being covalantly linked to the γ-carbon of the glutamine residue.
- an aminosaccharide or aminopolysaccharide, the aminogroup thereof being covalently linked to the δ-carbon of the glutamine residue.
- a saccharide, polysaccharide or alcohol, a hydroxygroup thereof being covalently linked to the δ-carbon of the glutamine residue.

12. Peptide or protein according to claim 11, wherein the glutamine comprising peptide or protein and the substrate are as defined in any of the claims 2-7.

13. Use of transglutaminase for the manufacture of a peptide or protein according to claims 11 or 12, the transaminase preferably being of microbial origin, preferably from a *Streptoverticillium sp.,* more preferably from *Streptoverticillium mobaraense*.

14. Use of transglutaminase for masking an epitope of a peptide or protein.
